# EUROPEAN PATENT APPLICATION

(11) **EP 4 442 678 A1**
(43) Date of publication of application: **09.10.2024**
(21) Application number: 23877744.5
(22) Date of filing: 13.10.2023
(51) Int. Cl.: C07C 219/20, A01N 37/44

(54) **COMPOUND AND MOLDED BODY COMPRISING SAME**

(30) Priority: 13.10.2022 KR 20220131730; 12.10.2023 KR 20230136265
(71) Applicant: LG Chem, Ltd., Yeongdeungpo-gu Seoul 07336 (KR)
(72) Inventor: SEO, Ue Ryung, Daejeon 34122 (KR); KANG, Soonhee, Daejeon 34122 (KR); LEE, Ji Seok, Daejeon 34122 (KR); CHOI, Hyungsam, Daejeon 34122 (KR)
(74) Representative: Plasseraud IP
(86) International application number: PCT/KR2023/015813
(87) International publication number: WO 2024/080821

(57) **Abstract**

The present specification relates to a compound represented by Chemical Formula 1 and dissolved in at least two of ether, toluene and ethyl acetate at room temperature, and a molded article including the same.

## Description

### [Technical Field]

The present specification relates to a compound and a molded article including the same.

This application claims priority to and the benefit of Korean Patent Application No. 10-2022-0131730 filed in the Korean Intellectual Property Office on October 13, 2022 and Korean Patent Application No. 10-2023-0136265 filed in the Korean Intellectual Property Office on October 12, 2023, the entire contents of which are incorporated herein by reference.

### [Background Art]

Recently, various products such as daily supplies or hygiene products are required to have high antibacterial properties.

The degree of antibacterial properties required and the material requirements for imparting antibacterial properties differ depending on the material of the product requiring antibacterial properties and the state of final use. For example, the properties of the material for imparting antibacterial properties and the degree of antibacterial properties vary depending on the amount of antibacterial material applied to a product used and the materials used together.

However, in introducing antibacterial agents and the like, which inhibit bacterial proliferation into resins, it has not been easy to select and introduce an antibacterial component that is harmless to the human body, satisfies economic feasibility, and does not degrade the basic physical properties of a polymer resin, while exhibiting excellent bacterial proliferation inhibiting properties.

As one example, widely used quaternary ammonium-based antibacterial agents are mostly in the form of antibacterial agents substituted with halogen anions. However, in this case, there is a problem in that the antibacterial agent is dissolved only in a specific solvent, thereby reducing its practicality, and there is also a problem in that consumers have a negative perception of the antibacterial agent including a halogen.

Accordingly, there is a need for developing a material that can be dissolved in various solvents, exhibits high antibacterial properties, does not release antibacterial materials, and thus is harmless to the human body.

### [Citation List] (Patent Document 1) Korean Patent Application Laid-Open No. 10-2009-0131847

### [Detailed Description of the Invention]

### [Technical Problem]

The present specification provides a compound and a molded article including the same.

### [Technical Solution]

An exemplary embodiment of the present specification provides a compound represented by the following Chemical Formula 1, and
dissolved in at least two of ether, toluene and ethyl acetate at room temperature.

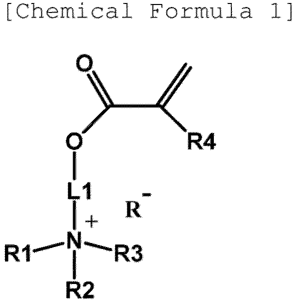

In Chemical Formula 1,
L1 is an alkylene group,
R1 to R3 are the same as or different from each other, and are each independently an alkyl group, and at least one of R1 to R3 is an alkyl group having 5 to 30 carbon atoms,
R4 is hydrogen or an alkyl group, and
R⁻ is a salicylate-based anion, a sulfate-based anion, a bisulfate-based anion, a benzoate-based anion, a sorbate-based anion, or a lactate-based anion.

Another exemplary embodiment of the present specification provides an antibacterial composition including the above-described compound.

Still another exemplary embodiment of the present specification provides a molded article including the above-described compound or prepared therefrom.

### [Advantageous Effects]

A compound according to some exemplary embodiments of the present specification can be dissolved in various solvents.

The compound according to some exemplary embodiments of the present specification has hydrophilic and hydrophobic properties, and thus has excellent antibacterial properties.

The compound according to some exemplary embodiments of the present specification can solve safety problems due to the release of antibacterial materials.

The compound according to some exemplary embodiments of the present specification can exhibit antibacterial properties within a short period of time.

Since the compound according to some exemplary embodiments of the present specification has little change in antibacterial activity depending on the amount of antibacterial material used, antibacterial properties can be exhibited within an expected range even when the unevenness of concentration occurs unintentionally during application to a product. Therefore, antibacterial properties are controlled within a specific range, so that excellent antibacterial properties can be safely imparted.

The compound according to some exemplary embodiments of the present specification has low toxicity, and thus can solve safety problems.

### [Brief Description of Drawings]

FIG. 1 is a view illustrating the NMR measurement results of Compound 12 in which R⁻ is Br⁻.
FIG. 2 is a view illustrating the NMR measurement results of Compound 13 in which R- is Br⁻.
FIG. 3 is a view illustrating the NMR measurement results of Compound 13 in which R⁻ is salicylate.
FIG. 4 is a view illustrating the NMR measurement results of Compound 13 in which R⁻ is citrate.
FIG. 5 is a view illustrating the NMR measurement results of Compound 13 in which R- is sorbate.
FIG. 6 is a view illustrating the NMR measurement results of Compound 13 in which R- is benzoate.
FIG. 7 is a view illustrating the NMR measurement results of Compound 12 in which R⁻ is sulfate.
FIG. 8 is a view illustrating the NMR measurement results of Compound 12 in which R⁻ is bisulfate.
FIG. 9 is a view illustrating the TGA measurement results.

### [Best Mode]

Hereinafter, the present specification will be described in detail.

An exemplary embodiment of the present specification provides a compound represented by the following Chemical Formula 1, and
dissolved in at least two of ether, toluene and ethyl acetate at room temperature.

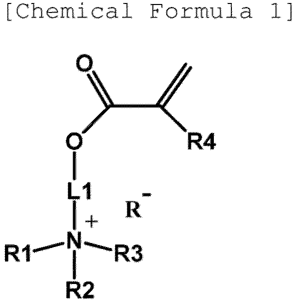

In Chemical Formula 1,
L1 is an alkylene group,
R1 to R3 are the same as or different from each other, and are each independently an alkyl group, and at least one of R1 to R3 is an alkyl group having 5 to 30 carbon atoms,
R4 is hydrogen or an alkyl group, and
R⁻ is a salicylate-based anion, a sulfate-based anion, a bisulfate-based anion, a benzoate-based anion, a sorbate-based anion, or a lactate-based anion.

In the related art, as quaternary ammonium-based antibacterial agents, antibacterial agents substituted with halogen anions have been used. However, in this case, there was a problem in that the antibacterial agent is dissolved only in a specific solvent, thereby reducing its practicality. Specifically, quaternary ammonium-based compounds in which halogen anions (F⁻, Cl⁻, Br⁻ or I⁻) in the related art are substituted, particularly, compounds represented by Chemical Formula 1 above, in which R⁻ is a halogen anion, are dissolved only in a specific solvent such as ethanol, methylene chloride, and acetone, but are not dissolved in ether, toluene, ethyl acetate, and the like, so that the compounds have a limitation in terms of their applicability, and thus have a problem in that the compounds cannot be used in lacquer paints, synthetic resin paints, acrylic paints, and the like. In addition, due to problems such as harmful effects such as toxicity of antibacterial agents including a halogen, the possibility of inducing environmental pollution, and caution in handling due to high reactivity, there is a tendency toward stricter regulations by authorities, and there is also a problem in that consumers have negative perceptions.

In contrast, the compound according to the present specification includes other specific anions without including a halogen group, and thus can be dissolved in various solvents, so that there is an advantage in that practicality is improved. Specifically, by changing anions to types other than halogen anions, the compound represented by Chemical Formula 1 according to the present specification exhibits an effect of being dissolved in various solvents in which the compound is not dissolved when the halogen anions are used. Therefore, by selectively using a solvent as necessary, the compound exhibits an effect in which it is possible to diversify the fields of application of the compound.

When one part "includes" one constituent element in the present specification, unless otherwise specifically described, this does not mean that another constituent element is excluded, but means that another constituent element may be further included.

In the present specification, among physical properties, physical properties which are affected by temperature are physical properties measured at room temperature, unless otherwise specified.

In the present specification, "room temperature" means the natural temperature at which a system has not been heated or cooled, for example, any one temperature within a range of about 10°C to 30°C, for example, a temperature of about 15°C, about 18°C, about 20°C, about 23°C, or about 25°C. Further, in the present specification, the unit of temperature is °C, unless otherwise specified.

In the present specification, when pressure among physical properties affects the results, the corresponding physical property is a physical property measured at normal pressure, unless otherwise specified.

In the present specification, "normal pressure" is the natural pressure under which a system has not been pressurized or depressurized, and refers to a pressure which is typically about 1 atmosphere (about 700 to 800 mmHg) .

In the present specification, when humidity among physical properties affects the results, the corresponding physical property is a physical property measured at a humidity that is not particularly controlled in the room temperature and normal pressure state, unless otherwise specified.

In the present specification, an "alkyl group" may be straight-chained or branched, and the number of carbon atoms thereof is not particularly limited, but is preferably 1 to 60. In an exemplary embodiment of the present specification, the number of carbon atoms of the alkyl group is 1 to 30. Specific examples of the alkyl group include a methyl group, an ethyl group, a propyl group, an isopropyl group, a butyl group, an isobutyl group, a tert-butyl group, a pentyl group, a hexyl group, a heptyl group, an octyl group, a nonyl group, a decyl group, an undecyl group, a dodecyl group, a tridecyl group, and the like, but are not limited thereto.

In the present specification, an "alkylene group" means a group having two bonding positions in an alkyl group, that is, a divalent group. The above-described description on the alkyl group may be applied to the alkylene group, except for a divalent alkylene group.

In the present specification, the "aryl group" may be monocyclic or polycyclic, and the number of carbon atoms thereof is not particularly limited, but is preferably 6 to 30. Specific examples of the aryl group include a phenyl group, a biphenyl group, a terphenyl group, a naphthyl group, an anthracenyl group, a phenanthryl group, a triphenylene group, and the like, but are not limited thereto.

In the present specification, being dissolved means that the solubility measured by the following Method 2 is 3 g/L or more.

### [Method 2]

0.2 g of a target to be measured is collected, and the total weight is allowed to be 6.6 g by adding a solvent thereto. After the mixture is stirred at 25°C for 30 minutes, an undissolved residual solute is removed. The amount of residual solute removed is measured, the amount of solute dissolved in the solvent is measured, and the measured amount is converted into a value for 1 L of solvent to evaluate solubility. In the above procedure, the residual solute is removed by filtering the solution with a sieve having a pore size of 0.45 um.

In an exemplary embodiment of the present specification, the compound is dissolved in two of ether, toluene, and ethyl acetate at room temperature.

In an exemplary embodiment of the present specification, the compound is dissolved in all three of ether, toluene, and ethyl acetate at room temperature.

In an exemplary embodiment of the present specification, the compound has a solubility in ether of 3 g/L or more at room temperature. Specifically, the solubility is 5 g/L or more, or 10 g/L or more. The upper limit of the solubility is not particularly limited, and for example, the solubility is 100 g/L or less, 90 g/L or less, or 80 g/L or less.

In an exemplary embodiment of the present specification, the compound has a solubility in toluene of 3 g/L or more at room temperature. Specifically, the solubility is 5 g/L or more, or 10 g/L or more. The upper limit of the solubility is not particularly limited, and for example, the solubility is 100 g/L or less, 90 g/L or less, or 80 g/L or less.

In an exemplary embodiment of the present specification, the compound has a solubility in ethyl acetate of 3 g/L or more at room temperature. Specifically, the solubility is 5 g/L or more, or 10 g/L or more. The upper limit of the solubility is not particularly limited, and for example, the solubility is 100 g/L or less, 90 g/L or less, or 80 g/L or less.

In the present specification, the solubility of 3 g/L or more means that 3 g or more of a compound is dissolved in 1 L of a solvent.

In an exemplary embodiment of the present specification, the compound is also dissolved in ethanol, methylene chloride, and acetone (ACT) at room temperature.

In an exemplary embodiment of the present specification, L1 is an alkylene group having 1 to 10 carbon atoms.

In an exemplary embodiment of the present specification, L1 is an alkylene group having 1 to 5 carbon atoms.

In an exemplary embodiment of the present specification, L1 is a methylene group; an ethylene group; a propylene group; or a butylene group.

In an exemplary embodiment of the present specification, any one of R1 to R3 is an alkyl group having 5 to 30 carbon atoms, and the others are the same as or different from each other, and are each independently an alkyl group having 1 to 30 carbon atoms.

In an exemplary embodiment of the present specification, any one of R1 to R3 is an alkyl group having 5 to 30 carbon atoms, and the others are the same as or different from each other, and are each independently an alkyl group having 1 to 20 carbon atoms.

In an exemplary embodiment of the present specification, any one of R1 to R3 is an alkyl group having 5 to 30 carbon atoms, and the others are the same as or different from each other, and are each independently an alkyl group having 1 to 10 carbon atoms.

In an exemplary embodiment of the present specification, L1 is an alkylene group having 1 to 10 carbon atoms, any one of R1 to R3 is an alkyl group having 5 to 30 carbon atoms, and the others of R1 to R3 are the same as or different from each other, and are each independently an alkyl group having 1 to 30 carbon atoms.

In an exemplary embodiment of the present specification, L1 is an alkylene group having 1 to 5 carbon atoms, any one of R1 to R3 is an alkyl group having 5 to 30 carbon atoms, and the others of R1 to R3 are the same as or different from each other, and are each independently an alkyl group having 1 to 30 carbon atoms.

In an exemplary embodiment of the present specification, L1 is a methylene group, an ethylene group, or a butylene group, any one of R1 to R3 is an alkyl group having 5 to 30 carbon atoms, and the others of R1 to R3 are the same as or different from each other, and are each independently an alkyl group having 1 to 30 carbon atoms.

In an exemplary embodiment of the present specification, two or more of R1 to R3 are an alkyl group having 6 to 30 carbon atoms, or the difference in carbon number between an alkyl group having the largest number of carbon atoms and an alkyl group having the smallest number of carbon atoms among R1 to R3 is 4 or more.

Specifically, three of R1 to R3 are an alkyl group having 6 to 30 carbon atoms; two of R1 to R3 are an alkyl group having 6 to 30 carbon atoms, the other one is an alkyl group having 1 to 30 carbon atoms; or the difference in carbon number between an alkyl group having the largest number of carbon atoms and an alkyl group having the smallest number of carbon atoms among R1 to R3 is 4 or more. In this case, an effect in which antibacterial activity is excellent is exhibited.

In an exemplary embodiment of the present specification, a difference in the number of carbon atoms between the alkyl group having the largest number of carbon atoms and the alkyl group having the smallest number of carbon atoms of 4 or more means that the asymmetry is large, and the difference in the number of carbon atoms may be 4 to 30, 5 to 30, or 5 to 10.

In an exemplary embodiment of the present specification, the compound is any one of the following structures.

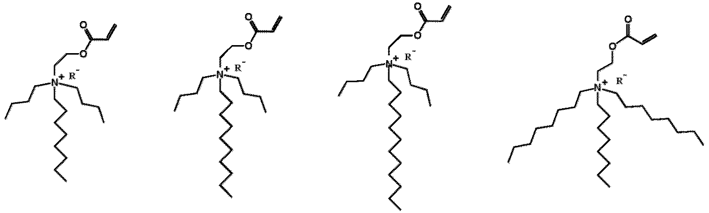

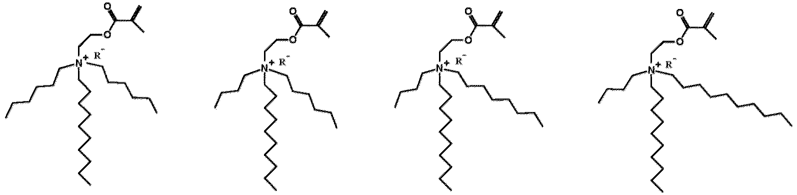

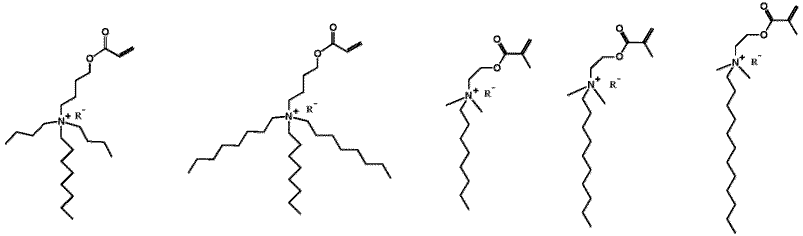

In the structures, R⁻ is the same as that described above.

In an exemplary embodiment of the present specification, the salicylate-based anion is represented by 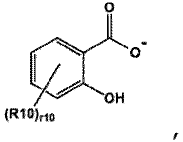 R10 is hydrogen; an alkyl group; or an aryl group, and r10 is an integer from 1 to 4.

In an exemplary embodiment of the present specification, when R⁻ in Chemical Formula 1 above is a salicylate-based anion, the compound exhibits solid properties.

In an exemplary embodiment of the present specification, R10 is hydrogen.

In an exemplary embodiment of the present specification, the benzoate-based anion is represented 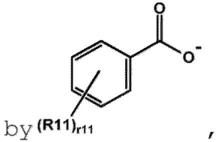 R11 is hydrogen; an alkyl group; or an aryl group, and r11 is an integer from 1 to 5.

In an exemplary embodiment of the present specification, when R⁻ in Chemical Formula 1 above is a benzoate-based anion, the compound exhibits liquid properties.

In an exemplary embodiment of the present specification, R11 is hydrogen.

In an exemplary embodiment of the present specification, the sorbate-based anion is represented

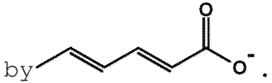

In an exemplary embodiment of the present specification, when R⁻ in Chemical Formula 1 above is a sorbate-based anion, the compound exhibits solid properties.

In an exemplary embodiment of the present specification, the lactate-based anion is represented by

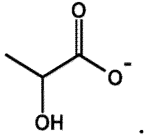

In an exemplary embodiment of the present specification, when R⁻ in Chemical Formula 1 above is a lactate-based anion, the compound exhibits solid properties.

In an exemplary embodiment of the present specification, the sulfate-based anion is represented by 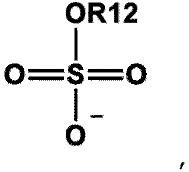 and R12 is an alkyl group.

In an exemplary embodiment of the present specification, when R⁻ in Chemical Formula 1 above is a sulfate-based anion, the compound exhibits solid properties.

In an exemplary embodiment of the present specification, the bisulfate-based anion is represented by 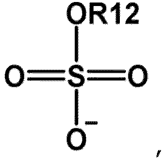 and R12 is hydrogen.

In an exemplary embodiment of the present specification, when R- in Chemical Formula 1 above is a bisulfate-based anion, the compound exhibits solid properties.

In the present specification, the alkyl group may mean an alkyl group having 1 to 30 carbon atoms as long as the number of carbon atoms thereof is not particularly limited, the aryl group may mean an aryl group having 6 to 30 carbon atoms as long as the number of carbon atoms thereof is not particularly limited, and the substituents thereof are those known in the art and are not particularly limited as long as they do not depart from the spirit of the present invention.

As described above, the properties of the compound vary depending on the type of anionic group. That is, in an exemplary embodiment of the present specification, properties of the compound can be regulated through anion substitution. Therefore, the compound according to an exemplary embodiment of the present specification exhibits an effect in which it is possible to diversify the fields of application of the compound by selectively substituting anions as necessary.

In an exemplary embodiment of the present specification, R⁻ is any one of the following structures.

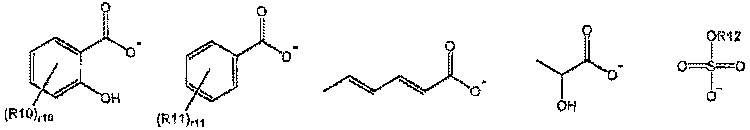

In the structures,
R10 to R12 are the same as or different from each other, and are each independently hydrogen; an alkyl group; or an aryl group,
r10 is an integer from 1 to 4, and when r10 is 2 or higher, two or more R10's are the same as or different from each other, and
r11 is an integer from 1 to 5, and when r11 is 2 or higher, two or more R11' are the same as or different from each other.

In an exemplary embodiment of the present specification, the compound is not precipitated even after a certain period of time has passed, and is maintained in a state of being dissolved in a solvent. That is, the compound exhibits dissolution stability.

In an exemplary embodiment of the present specification, the compound has antibacterial properties.

In the present specification, having antibacterial properties means that the antibacterial activity measured based on the following Method 1, in other words, the bacteriostatic reduction rate is 90% or more.

In an exemplary embodiment of the present specification, the compound has a bacteriostatic reduction rate of 90% or more against at least one strain selected from the group consisting of Gram-positive bacteria, Gram-negative bacteria, and molds, as measured by the following Method 1.

### [Method 1]

After 20 ml of a broth type medium (Nutrient broth, BD DIFCO., 8 g/L) inoculated with 3000 CFU/ml bacteria is transferred to a 50-ml conical tube, 0.005 g, 0.01 g, 0.015 g, or 0.020 g of a compound is added thereto, and the resulting mixture is vortexed. The well-mixed solution is cultured in a shaking water bath maintained at 35°C for 16 hours. After the cultured solution is diluted to 1/5 using a 1X PBS buffer solution, absorbance at a wavelength of 600 nm is measured using a UV/Vis spectrophotometer. The measured absorbance is compared to that of a solution cultured without a bacteriostatic material, and the bacteriostatic reduction rate is calculated using the following equation. Bacterial proliferation reduction ratio(%) = (1- ASample/AReference) × 100
*A_{Sample}* = Absorbance of bacteriostatic material-containing medium solution
*A_{Reference}* = Absorbance of solution cultured using only medium and microorganisms

In the present specification, the bacteriostatic material of Equation 1 is the compound of Chemical Formula 1.

In the present specification the CFU (Colony Forming Unit) means a colony forming unit, and CFU/mL means the number of CFUs per mL.

When the bacteriostatic reduction rate was evaluated for the compound of the present specification using Method 1 above, only the case where the bacteriostatic reduction rate (antibacterial activity) was 920 or higher was observed. As a result, it could be confirmed that the compound according to the present specification has excellent antibacterial activity.

As used herein, the term Gram-positive bacteria is a general term for bacteria that are stained purple when stained using the Gram staining method, and Gram-positive bacteria exhibit a purple color without discoloration even though the Gram-positive bacteria are stained with a basic dye such as crystal violet and then treated with ethanol because the cell walls of Gram-positive bacteria are composed of several layers of peptidoglycan.

In an exemplary embodiment of the present specification, the Gram-positive bacteria are selected from *Enterococcus faecalis, Staphylococcus aureus, Streptococcus pneumoniae, Enterococcus faecium* and *Lactobacillus lactis.* Specifically, the Gram-positive bacteria are any one selected from the above-described examples, but are not limited thereto.

In the present specification, the Gram-negative bacteria are a general term for bacteria that are stained red when stained with the Gram staining method, and Gram-negative bacteria have an outer membrane composed of lipid polysaccharides, lipid proteins, and/or other complex polymeric materials instead of having a cell wall with a relatively small amount of peptidoglycan compared to Gram-positive bacteria.

In an exemplary embodiment of the present specification, the Gram-negative bacteria are selected from *Proteus mirabilis, Escherichia coli, Salmonella typhi, Pseudomonas aeruginosa* and *Vibrio cholerae.* Specifically, the Gram-negative bacteria are any one selected from the above-described examples, but are not limited thereto.

In an exemplary embodiment of the present specification, the mold may be *Candida albicans,* but is not limited thereto.

In an exemplary embodiment of the present specification, the compound has an antibacterial activity of 90% or more against Gram-positive bacteria as measured by Method 1.

In an exemplary embodiment of the present specification, the compound has an antibacterial activity of 90% or more against Gram-negative bacteria as measured by Method 1.

Since the strains of the Gram-positive bacteria, Gram-negative bacteria and molds may not only induce various diseases upon contact, but also cause secondary infections, it is preferred to exhibit antibacterial properties against all of the Gram-positive bacteria, Gram-negative bacteria, and molds using one compound.

In an exemplary embodiment of the present specification, the compound has an acute oral toxicity dose LD50 of more than 300 mg/Kg, preferably 320 mg/Kg or more. The higher the acute oral toxicity dose LD50 of the compound according to the above-described exemplary embodiments, the lower the toxicity, so that the high acute oral toxicity dose is advantageous. However, the value of LD50 may be determined from the viewpoint that the above-described antibacterial activity needs also be satisfied. For example, the above-described compound may have an acute oral toxicity dose LD50 of 50,000 mg/Kg or less, for example, 10,000 mg/Kg or less, 5,000 mg/Kg or less, or 2,000 mg/Kg or less. In an example, the compound according to the above-described exemplary embodiments may have an acute oral toxicity dose LD50 of 1,000 mg/Kg or less.

An exemplary embodiment of the present specification provides an antibacterial composition including the above-described compound.

In an exemplary embodiment of the present specification, the above-described antibacterial composition further includes a solvent, and the solvent is at least one of ether, toluene, ethyl acetate, acetone, water, acetonitrile, ethanol, dichloromethane, chloroform, and hexane.

In an exemplary embodiment of the present specification, the solvent included in the antibacterial composition is one to ten, one to seven, one to five, one to three, or one of the above-described solvents.

In an exemplary embodiment of the present specification, the solvent included in the antibacterial composition is one to three of ether, toluene, and ethyl acetate.

In an exemplary embodiment of the present specification, the solvent included in the antibacterial composition is one of ether, toluene, and ethyl acetate.

An exemplary embodiment of the present specification provides a molded article including the above-described antibacterial resin or prepared therefrom. The molded article may be specifically an automobile part, a blow molding molded article, an inflation molded article, a cast molded article, an extrusion laminate molded article, an extrusion molded article, a foam molded article, an injection molded article, a sheet, a film, a fiber, a monofilament, or a non-woven fabric, but is not limited to the examples. The automobile part is an interior and exterior material for automobiles.

### [Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below. The Examples of the present specification are provided to more completely explain the present specification to a person with ordinary skill in the art.

### <Preparation Example 1>

### Preparation Example 1-1. Synthesis of Compound 1

### Step 1.

(1) 0.1 mol of 2- (dibutylamino) ethanol (DBAE), 0.1 mol of trimethylamine and 0.001 mol of hydroquinone were added to 100 mL of THF (solvent).
(2) 0.1 mol of acryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

### Step 2.

(1) The product of Step 1 and 1-bromooctane were dissolved at 50 wt% in acrylonitrile (solvent) at a molar ratio of 1:1.
(2) Subsequently, p-methoxyphenol, which is a polymerization inhibitor, was added (ratio with reaction product is 1:0.001 (eq)).
(3) The resulting mixture was reacted at 50°C for 20 hours.
(4) After static precipitation (MTBE:reaction solution = 15:1 (volume ratio)) in methyl t-butyl ether (MTBE), the mixture was filtered.
(5) The resulting product was dried under vacuum at 45°C.

### Step 3.

(1) The product of Step 2 was dissolved in water.
(2) A salt was dissolved in water.
(3) The two solutions prepared in (1) and (2) were mixed and stirred overnight.
(4) After the reaction was completed, Compound 1 was prepared by extracting the organic layer using ethyl acetate.

As the salt used in Step 3, sodium salicylate, sodium citrate, or sodium benzoate was used depending on the type of compound to be prepared.

### Preparation Example 1-2. Synthesis of Compounds 2 and 3

Compounds 2 and 3 were prepared in the same manner as in Preparation Example 1-1, except that 1-bromodecane (preparation of Compound 2) or 1-bromododecane (preparation of Compound 3) was used instead of 1-bromooctane in (1) of Step 2 of Preparation Example 1-1.

### Preparation Example 1-3. Synthesis of Compound 4

Compound 4 was prepared in the same manner as in Preparation Example 1-1, except that 2-(dioctylamino)ethanol (DOAE) was used instead of 2-(dibutylamino)ethanol in (1) of Step 1 of Preparation Example 1-1.

### Preparation Example 1-4. Synthesis of Compound 5

### Step 1.

(1) 0.1 mol of 2-(dihexylamino)ethanol (DHAE), 0.1 mol of trimethylamine and 0.001 mol of hydroquinone were put into 100 mL of THF (solvent).
(2) 0.1 mol of methacryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

### Step 2.

(1) The product of Step 1 and 1-bromodecane were dissolved at 50 wt% in acrylonitrile (solvent) at a molar ratio of 1:1.
(2) Subsequently, p-methoxyphenol, which is a polymerization inhibitor, was added (ratio with reaction product is 1:0.001 (eq)).
(3) The resulting mixture was reacted at 50°C for 20 hours.
(4) After static precipitation (MTBE:reaction solution = 15:1 (volume ratio)) in methyl t-butyl ether (MTBE), the mixture was filtered.
(5) The resulting product was dried under vacuum at 45°C.

### Step 3.

(1) The product of Step 2 was dissolved in water.
(2) A salt was dissolved in water.
(3) The two solutions prepared in (1) and (2) were mixed and stirred overnight.
(4) After the reaction was completed, Compound 5 was prepared by extracting the organic layer using ethyl acetate.

As the salt used in Step 3, sodium salicylate, sodium citrate, or sodium benzoate was used depending on the type of compound to be prepared.

### Preparation Example 1-5. Synthesis of Compound 6

Compound 6 was prepared in the same manner as in Preparation Example 1-4, except that 2-(butylhexylamino)ethanol (BHAE) was used instead of 2-(dihexylamino)ethanol in (1) of Step 1 of Preparation Example 1-4.

### Preparation Example 1-6. Synthesis of Compound 7

Compound 7 was prepared in the same manner as in Preparation Example 1-4, except that 2-(butyloctylamino)ethanol (BOAE) was used instead of 2-(dihexylamino)ethanol in (1) of Step 1 of Preparation Example 1-4.

### Preparation Example 1-7. Synthesis of Compound 8

Compound 8 was prepared in the same manner as in Preparation Example 1-4, except that 2-(butyldecylamino)ethanol (BOAE) was used instead of 2-(dihexylamino)ethanol in (1) of Step 1 of Preparation

### Example 1-4.

### Preparation Example 1-8. Synthesis of Compound 9

Compound 9 was prepared in the same manner as in Preparation Example 1-1, except that 2-(dibutylamino)butanol (DBAB) was used instead of 2-(dibutylamino)ethanol in (1) of Step 1 of Preparation Example 1-1.

### Preparation Example 1-9. Synthesis of Compound 10

Compound 10 was prepared in the same manner as in Preparation Example 1-1, except that 2-(dioctylamino)butanol (DOAB) was used instead of 2-(dibutylamino)ethanol in (1) of Step 1 of Preparation Example 1-1.

### Preparation Example 1-10. Synthesis of Compound 11

### Step 1.

(1) 5.3 g of 1-bromooctane, 3.7 g of 2-(dimethylamino)ethyl methacrylate, 89.4 mg of 4-methoxyphenol, and 12 mL of acetonitrile were put into a two-neck round bottom flask (RBF).
(2) The resulting mixture was reacted at 60°C for 6 hours.
(3) After static precipitation (MTBE:reaction solution = 15:1 (volume ratio)) in methyl t-butyl ether (MTBE), the mixture was filtered.
(4) The resulting product was dried under vacuum at 45°C.

### Step 2.

(1) The product of Step 1 was dissolved in water.
(2) A salt to be substituted was dissolved in water.
(3) The two solutions prepared in (1) and (2) were mixed and stirred overnight.
(4) After the reaction was completed, Compound 11 was prepared by extracting the organic layer using ethyl acetate.

As the salt, sodium salicylate, sodium citrate, sodium benzoate, sodium sorbate, or sodium bisulfate was used depending on the type of compound to be prepared.

However, when sodium dodecyl sulfate was used as the salt to be substituted, the reaction was completed in (4) of Step 2, and then an excess amount of sodium chloride was added to extract the compound (salting out).

### Preparation Example 1-11. Synthesis of Compounds 12 and 13

Compounds 12 and 13 were prepared in the same manner as in Preparation Example 1-10, except that 1-bromodecane (preparation of Compound 12) or 1-bromododecane (preparation of Compound 13) was used instead of 1-bromooctane in Preparation Example 1-10.

Compounds 12 and 13 in which R⁻ is Br⁻ were obtained by terminating the reaction in Step 1 of Preparation Examples 1-10 and 1-11 above.

Although a static precipitation method of adding a reactant to a nonsolvent was used in Preparation Examples 1-1 to 1-11, a reverse precipitation method of adding a nonsolvent to a reactant may also be used. Furthermore, in addition to 15:1, other ratios of MTBE and reaction solution may be used, and for example, 12:1 and 26:1 may be used.

The structures of Compounds 1 to 13 prepared in Preparation Examples 1-1 to 1-11 above are as follows. 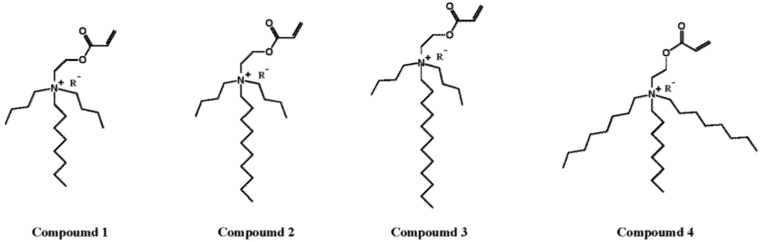 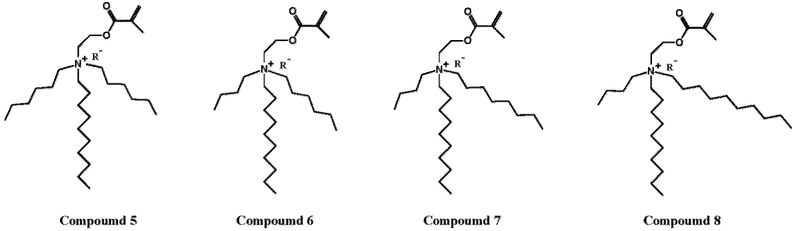 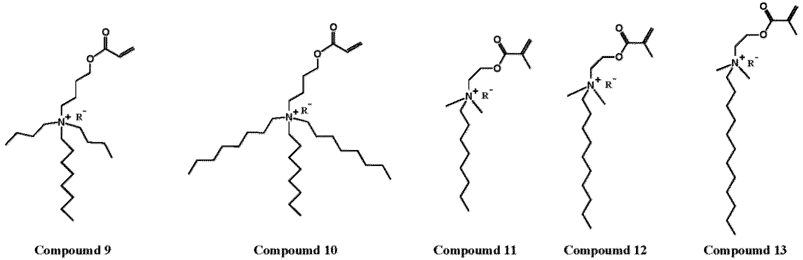 * R-: salicylate 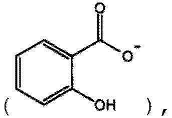 citrate 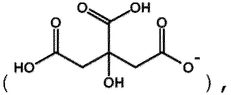 benzoate 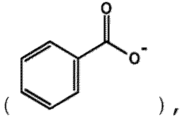 sorbate 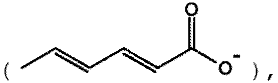 sulfate 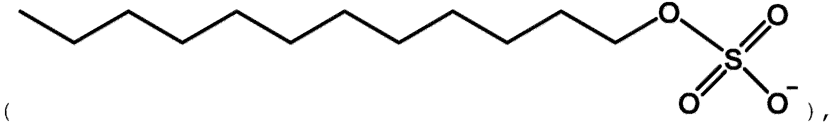 or bisulfate

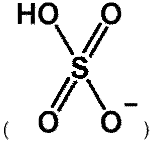

Through NMR measurement, it was confirmed that Compounds 1 to 13 substituted with specific anions were synthesized.

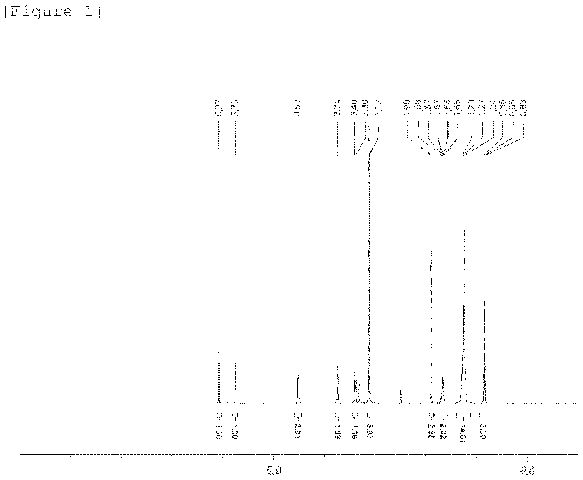

FIG. 1 illustrates the NMR measurement results of Compound 12 in which R⁻ is Br⁻.

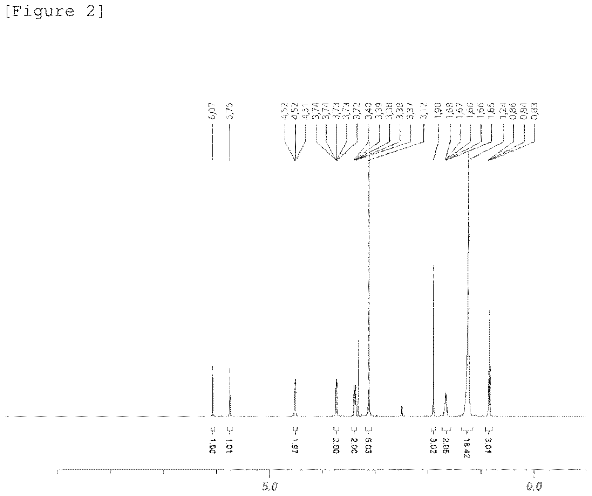

FIG. 2 illustrates the NMR measurement results of Compound 13 in which R⁻ is Br⁻.

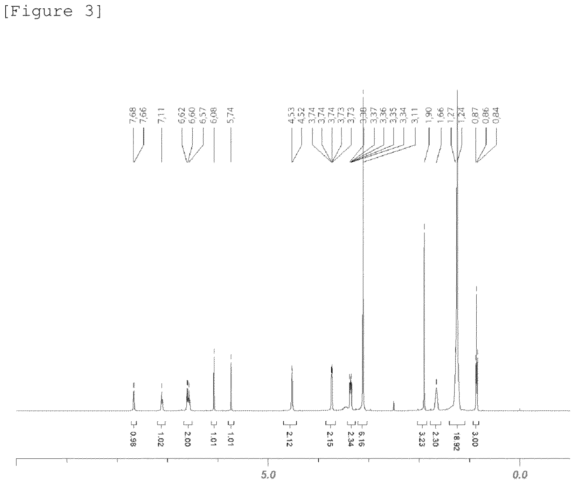

FIG. 3 illustrates the NMR measurement results of Compound 13 in which R⁻ is salicylate.

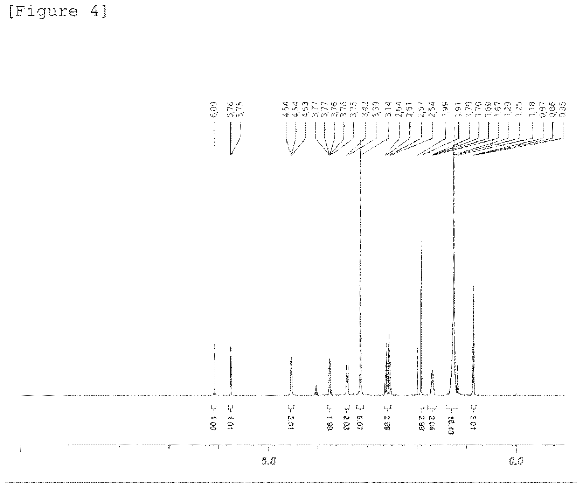

FIG. 4 illustrates the NMR measurement results of Compound 13 in which R⁻ is citrate.

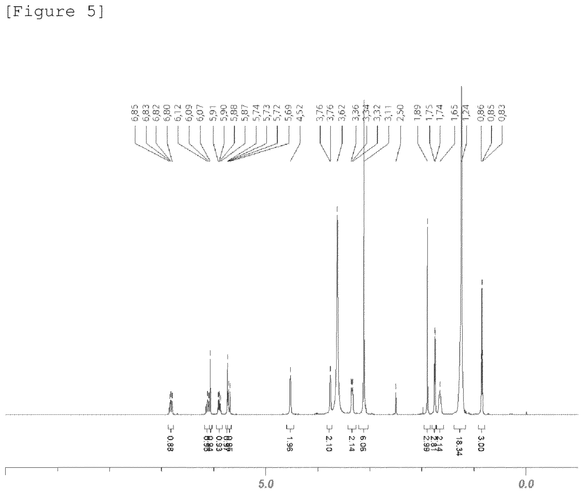

FIG. 5 illustrates the NMR measurement results of Compound 13 in which R⁻ is sorbate.

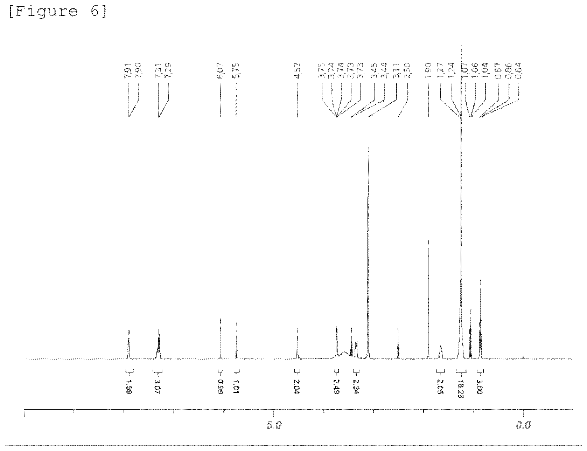

FIG. 6 illustrates the NMR measurement results of Compound 13 in which R⁻ is benzoate.

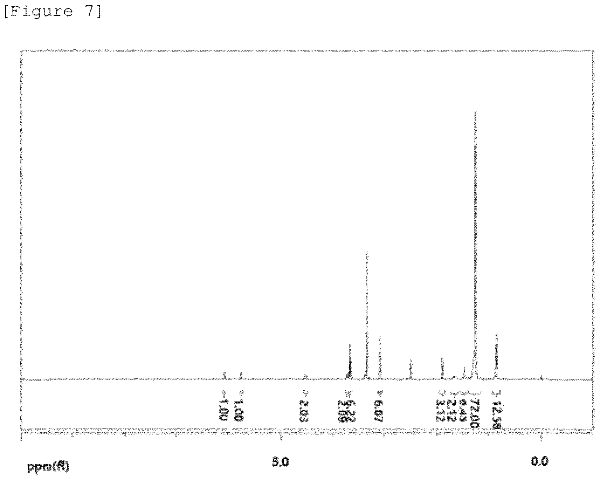

FIG. 7 illustrates the NMR measurement results of Compound 12 in which R⁻ is sulfate.

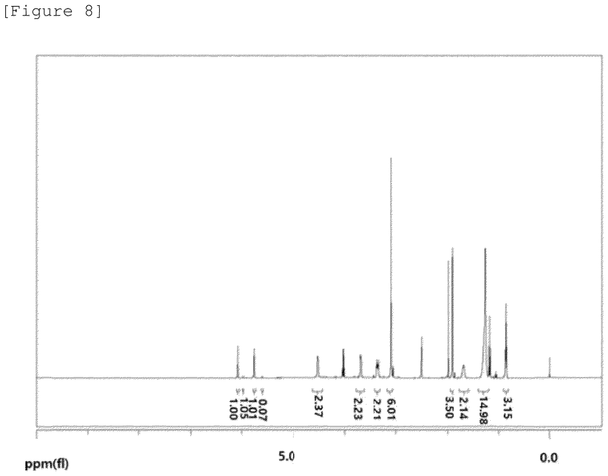

FIG. 8 illustrates the NMR measurement results of Compound 12 in which R⁻ is bisulfate.

### <Experimental Example 1> Measurement of solubility

0.2 g of a target to be measured was collected, and the total weight was allowed to be 6.6 g by adding a solvent thereto. After the mixture was stirred at 25°C for 30 minutes, an undissolved residual solute was removed. The amount of residual solute removed was measured, the amount of solute dissolved in the solvent was measured, and the measured amount was converted into a value for 1 L of solvent to evaluate solubility. In the above procedure, the residual solute was removed by filtering the solution with a sieve having a pore size of 0.45 um.

The target to be measured was Compound 13 prepared in Preparation Example 1-11 above, and the target was referred to as shown in the following Table 1 according to the type of R-.

The solubility measurement results are shown in the following Table 1.

**[Table 1]**

| No. | R- | Solubility | | | | | |
|---|---|---|---|---|---|---|---|
| | | Ethanol | Methylene chloride | Acetone | ether | toluene | Ethyl acetate |
| Comparative Example 1-1 | Br⁻ | O | O | O | X | X | X |
| Comparative Example 1-2 | Citrate | O | O | O | X | X | O |
| Example 1-1 | Salicylate | O | O | O | X | O | O |
| Example 1-2 | Benzoate | O | X | O | O | O | O |
| Example 1-3 | Sorbate | X | X | O | O | O | O |
| Example 1-4 | Sulfate | O | O | O | X | O | O |
| Example 1-5 | Bisulfate | O | O | O | O | O | O |

| | | | | | | | |
|---|---|---|---|---|---|---|---|
| - O: solubility 3 g/L or more - X: solubility less than 3 g/L | | | | | | | |

From Table 1 above, it can be confirmed that the compound according to an exemplary embodiment of the present specification can improve solubility in various solvents by including a specific anion other than a halogen group (specifically Br⁻) or citrate as an anion.

### <Experimental Example 2> Measurement of antibacterial activity

After 20 ml of a broth type medium (Nutrient broth, BD DIFCO., 8 g/L) inoculated with 3000 CFU/ml *E. coli* bacteria was transferred to a 50-ml conical tube, 0.005 g, 0.01 g, 0.015 g, or 0.020 g of a compound was added thereto, and the resulting mixture was vortexed. The well-mixed solution was cultured in a shaking water bath maintained at 35°C for 16 hours. After the cultured solution was diluted to 1/5 using a 1X PBS buffer solution, absorbance at a wavelength of 600 nm was measured using a UV/Vis spectrophotometer. The measured absorbance was compared to that of a solution cultured without a bacteriostatic material, and the bacteriostatic reduction rate was calculated using the following equation.

The target to be measured was Compound 13 prepared in Preparation Example 1-11 above, and the target was referred to as shown in the following Table 2 according to the type of R-. Bacterial proliferation reduction ratio%) = (1- ASample/AReference) × 100
*A_{Sample}* = Absorbance of bacteriostatic material-containing medium solution
*A_{Reference}* = Absorbance of solution cultured using only medium and microorganisms

The derived bacteriostatic reduction rate (antibacterial activity) is shown in the following Table 2.

**[Table 2]**

| No. | R⁻ | Antibacterial activity | | | |
|---|---|---|---|---|---|
| | | 0.5 phr | 1 phr | 1.5 phr | 2 phr |
| Comparative Example 2-1 | Br⁻ | 91.9 | 91.5 | 91.9 | 93.1 |
| Comparative Example 1-2 | Citrate | 92.9 | 92.8 | 92.9 | 93.2 |
| Example 2-1 | Salicylate | 95.7 | 95.7 | 95.8 | 95.7 |
| Example 2-2 | Benzoate | 96.1 | 96.2 | 96.1 | 96.3 |
| Example 2-3 | Sorbate | 95.1 | 95.3 | 95.3 | 95.7 |
| Example 2-5 | Sulfate | 95.2 | 95.8 | 95.7 | 96.1 |
| Example 2-6 | Bisulfate | 95.7 | 96.1 | 96.2 | 96.2 |

In Table 2 above, 0.5 phr, 1 phr, 1.5 phr and 2 phr are as follows, respectively - 0.5 phr: 0.005 g of the compound is added to 20 mL of 3000 CFU/mL bacterial solution
- 1 phr: 0.01 g of the compound is added to 20 mL of 3000 CFU/mL bacterial solution
- 1.5 phr: 0.015 g of the compound is added to 20 mL of 3000 CFU/mL bacterial solution
- 2 phr: 0.02 g of the compound is added to 20 mL of 3000 CFU/mL bacterial solution

From Table 2 above, it can be confirmed that the compound according to an exemplary embodiment of the present specification exhibits antibacterial activity equal to or higher than the compound including Br⁻ and the compound including citrate by using a specific anion.

### <Experimental Example 3> Evaluation of toxicity stability

The acute oral toxicity dose LD50 was measured by 3T3 Neutral Red Uptake (NRU) assay (OECD Guidance Document NO 129). Specifically, LD50 was calculated by the following method.

| | |
|---|---|
| **Determination of IC50 in cytotoxicity test** | |
| **↓ IC50** | |
| **Calculation of LD50 by applying linear regression model of cytotoxicity register** | |
| **IC50 is used for linear regression analysis** | |
| **log(LD50) = 0.433 × log(IC50) + 0.625** | |
| **↓ LD50** | |
| ***In vivo* acute toxicity test** | |
| **The calculated LD50 value is used as initial dose below** | |
| | **1. Up-and-down procedure(UDP)** |
| | **2. Fixed dose procedure(FDP)** |
| | **3. Acute toxic class method(ATC)** |

The calculated results are shown in the following Table 3.

The target to be measured was Compound 13 prepared in Preparation Example 1-11 above, and the target was referred to as shown in the following Table 3 according to the type of R-.

**[Table 3]**

| No. | R⁻ | LD50 (mg/kg) |
|---|---|---|
| Comparative Example 3-1 | Br⁻ | 518 |
| Comparative Example 3-2 | Citrate | 327 |
| Example 3-1 | Salicylate | 590 |
| Example 3-2 | Benzoate | 582 |
| Example 3-3 | Sorbate | 322 |

From Table 3 above, it can be confirmed that the compound according to an exemplary embodiment of the present specification has an LD50 belonging to Acute Toxicity Standard Category 4 (300 mg/kg to 2,000 mg/kg) in the same manner as when a halogen group is applied as an anion and when citrate is used as an anion. Therefore, from Tables 1 to 3 above, it can be confirmed that by using a specific anion, the compound according to an exemplary embodiment of the present specification has an acute toxicity standard that belongs to the equivalent Category 4, such as Br⁻ and citrate, the antibacterial activity is improved, and the types of solvents in which the compound is dissolved become more diverse.

### <Experimental Example 4> Evaluation of heat resistance

In this experiment, the evaluation of heat resistance was defined as an extrapolated intersection point (primary thermal decomposition temperature) between a starting mass reference line and the tangent line of the maximum slope point in the first mass reduction section of a mass loss curve measured by a thermogravimetric analyzer (TGA) in an N₂ atmospheric environment.

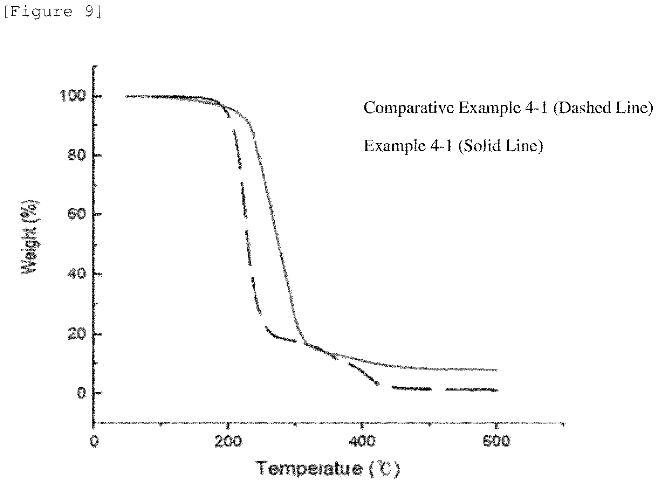

In the present invention, it is determined that when the primary thermal decomposition temperature of the compound has a value higher than the primary thermal decomposition temperature of the material before anion substitution, the heat resistance has increased. The measurement results are shown in the following FIG. 9.

The target to be measured was Compound 12 prepared in Preparation Example 1-11 above, and the target was shown in FIG. 9 as Comparative Example 4-1 and Example 4-1 according to the type of R⁻.

Referring to FIG. 9, it can be confirmed that the Td (thermal decomposition temperature) of Comparative Example 4-1 in which the anion is Br⁻ is about 230°C, which is higher than that in Example 4-1 in which the anion is substituted with bisulfate, and heat resistance is improved.

## Claims

1. A compound represented by the following Chemical Formula 1, and
dissolved in at least two of ether, toluene and ethyl acetate at room temperature: 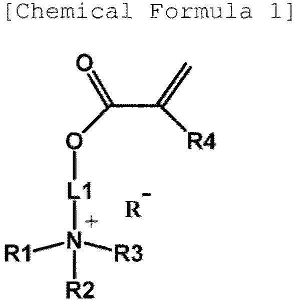
wherein, in Chemical Formula 1,
L1 is an alkylene group,
R1 to R3 are the same as or different from each other, and are each independently an alkyl group, and at least one of R1 to R3 is an alkyl group having 5 to 30 carbon atoms,
R4 is hydrogen or an alkyl group, and
R⁻ is a salicylate-based anion, a sulfate-based anion, a bisulfate-based anion, a benzoate-based anion, a sorbate-based anion, or a lactate-based anion.

2. The compound of claim 1, wherein R⁻ is any one of the following structures: 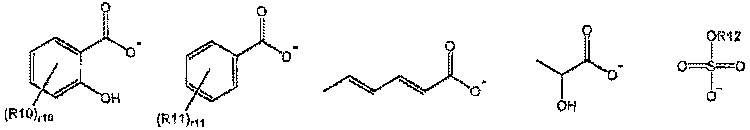
in the structures,
R10 to R12 are the same as or different from each other, and are each independently hydrogen; an alkyl group; or an aryl group,
r10 is an integer from 1 to 4, and when r10 is 2 or higher, two or more R10's are the same as or different from each other, and
r11 is an integer from 1 to 5, and when r11 is 2 or higher, two or more R11' are the same as or different from each other.

3. The compound of claim 1, wherein any one of R1 to R3 is an alkyl group having 5 to 30 carbon atoms, and the others are the same as or different from each other, and are each independently an alkyl group having 1 to 30 carbon atoms.

4. The compound of claim 1, wherein the compound is any one of the following structures: 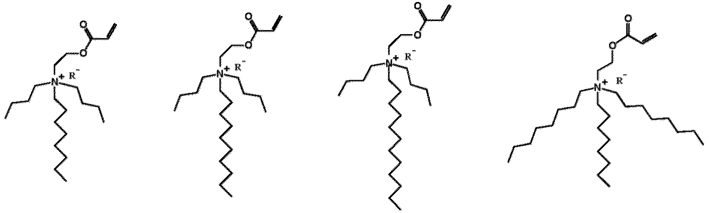 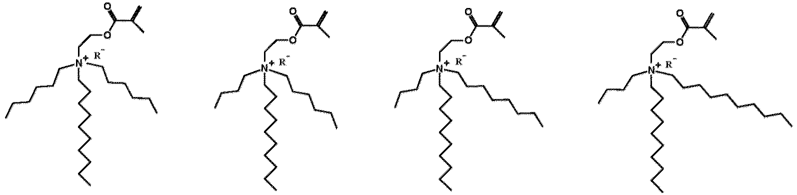 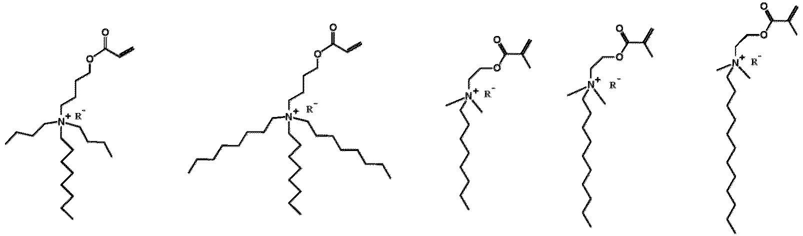 in the structures, R⁻ is the same as that defined in claim 1.

5. The compound of claim 1, wherein the compound has a bacteriostatic reduction rate of 90% or more against at least one strain selected from the group consisting of Gram-positive bacteria, Gram-negative bacteria, and molds, as measured by the following Method 1:
[Method 1]
After 20 ml of a broth type medium which is Nutrient broth, BD DIFCO., 8 g/L inoculated with 3000 CFU/ml bacteria is transferred to a 50-ml conical tube, 0.005 g, 0.01 g, 0.015 g, or 0.020 g of a compound is added thereto, and the resulting mixture is vortexed, the well-mixed solution is cultured in a shaking water bath maintained at 35°C for 16 hours, after the cultured solution is diluted to 1/5 using a 1X PBS buffer solution, absorbance at a wavelength of 600 nm is measured using a UV/Vis spectrophotometer, the measured absorbance is compared to that of a solution cultured without a bacteriostatic material, and the bacteriostatic reduction rate is calculated using the following equation. Bacterial proliferation reduction ratio%) = (1- ASample/AReference) × 100
*A_{Sample}* = Absorbance of bacteriostatic material-containing medium solution
*A_{Reference}* = Absorbance of solution cultured using only medium and microorganisms

6. The compound of claim 5, wherein the Gram-positive bacteria are selected from *Enterococcus faecalis, Staphylococcus aureus, Streptococcus pneumoniae, Enterococcus faecium* and *Lactobacillus lactis.*

7. The compound of claim 5, wherein the Gram-negative bacteria are selected from *Proteus mirabilis, Escherichia coli, Salmonella typhi, Pseudomonas aeruginosa* and *Vibrio cholerae.*

8. An antibacterial composition comprising the compound according to any one of claims 1 to 7.

9. The antibacterial composition of claim 8, further comprising a solvent,
wherein the solvent is at least one of ether, toluene, ethyl acetate, acetone, water, acetonitrile, ethanol, dichloromethane, chloroform, and hexane.

10. A molded article comprising the compound according to any one of claims 1 to 7 or prepared therefrom.
